# EUROPÄISCHE PATENTSCHRIFT

(11) **EP 0 516 251 B1**
(45) Veröffentlichungstag und Bekanntmachung des Hinweises auf die Patenterteilung: **11.09.1996**
(21) Anmeldenummer: 92250064.0
(22) Anmeldetag: 19.03.1992
(51) Int. Cl.: G01B 11/06, G01N 33/12, A61B 5/103

(54) **Vorrichtung zur Bestimmung der Dicke einer Fettschicht**
Device to detect the thickness of a layer of fat
Dispositif pour détecter l'épaisseur d'une couche de graisse

(30) Priorität: 26.04.1991 DE 4113749
(43) Veröffentlichungstag der Anmeldung: 02.12.1992
(73) Patentinhaber: Möller, Reinhard, Dr., A-8010 Graz (AT)
(72) Erfinder: Möller, Reinhard, Dr., A-8010 Graz (AT)
(74) Vertreter: Wildhack, Helmut, Dipl.-Ing. Dr.

(56) Entgegenhaltungen:
- DE-A- 2 518 518
- DE-A- 2 523 956
- GB-A- 1 549 065
- US-A- 3 435 242
- US-A- 5 014 713

## Beschreibung

Die Erfindung betrifft eine bekannte Vorrichtung gemäß dem Oberbegriff des Patentanspruches 1.

Aus der DE-A1-25 18 518 ist es nämlich bekannt, unter Verwendung von mindestens zwei Lichtquellen und einem Lichtsensor die Dicke einer Schicht zu bestimmen. Eine Vorrichtung zum Bestimmen einer Fettschicht durch Einstechen einer Beleuchtungs- und Sensoreinheit in die Schicht ist Gegenstand der DE-A1-25 23 956 bzw. der GB-PS 15 49 065.

Die Erfindung geht auch von einer Einrichtung aus, so wie sie in der nicht vorveröffentlichten US-PS-50 14 713 beschrieben ist.

Aus der US-PS-34 35 242 ist eine Einrichtung zur Durchstrahlungsuntersuchung von Materialien bekannt.

Der Erfindung liegt die Aufgabe zugrunde, eine Vorrichtung zu schaffen, die auf einfache und zuverlässige Weise die Bestimmung der Dicke einer Fettschicht ermöglicht.

Erfindungsgemäß wird diese Aufgabe durch die im kennzeichnenden Teil des Anspruches 1 angegebenen Merkmale gelöst. Die Ansprüche 2 bis 8 geben bevorzugte Ausgestaltungen der Vorrichtung an.

Die Erfindung wird im folgenden anhand einer Zeichnung erläutert. Dabei zeigt:
- Figur 1: schematisch eine Ausführungsform der erfindungsgemäßen Vorrichtung zum Bestimmen der Dicke einer Fettschicht.
- Figur 2: schematisch die Anordnung der Lichtquellen bzw. des Lichtsensors nach einem Ausführungsbeispiel der Erfindung und
- Figur 3: den zeitlichen Verlauf von Intensitätsmessungen nach einem Ausführungsbeispiel der Erfindung.

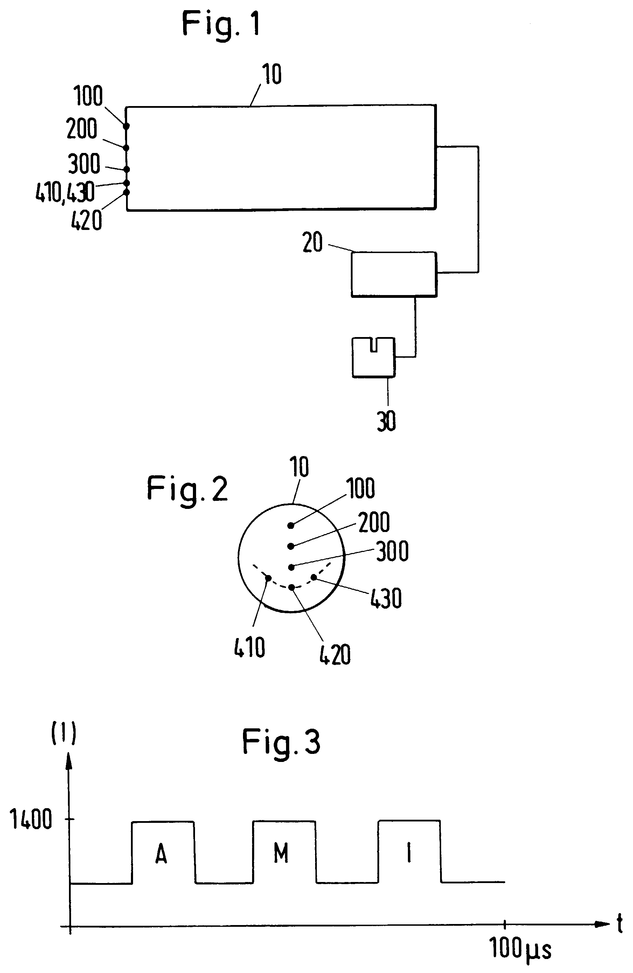

Gemäß Figur 1 umfaßt die erfindungsgemäße Vorrichtung eine Sonde 10, an der sich ein Lichtsensor 100 sowie Leuchtdioden 200, 300, 410, 420 und 430 befinden.

Die Sonde 10 ist mit einem Rechner 20 und dieser wiederum mit einem Kartenlese/Schreibgerät 30 verschaltet.

Figur 2 zeigt die Anordnung des Sensors 100 sowie der Leuchtdioden 200, 300, 410, 420 und 430 an der Stirnseite der Sonde 10.

Im folgenden ist die Betriebsweise und Anwendung der Vorrichtung erläutert.

Die Sonde 10 wird mit ihrer Stirnseite, auf der sich der Lichtsensor sowie die Leuchtdioden befinden an dem Körper angelegt. Sodann werden die Leuchtdioden nacheinander pulsförmig angesteuert. Zwischen den Pulsen zur Ansteuerung der Leuchtdioden liegen jeweils Dunkelzeiten.

Figur 3 zeigt den Verlauf der von dem Lichtsensor gemessenen Intensitäten. Wie Figur 3 zeigt, werden die äußeren (A) Leuchtdioden 410, 420, 430 gleichzeitig, danach die mittlere (M) Leuchtdiode 300 und danach die innere (I) Leuchtdiode 200 angesteuert, wobei vor der ersten Ansteuerung, zwischen den jeweiligen Ansteuerungen sowie nach der letzten Ansteuerung sogenannte "Dunkelzeiten" liegen, um eine Kalibrierung zu erlauben.

In diesem Ausführungsbeispiel ist das Gerät auf eine Schichtdicke von 14 mm normiert. Bei dieser Schichtdicke sind die Intensitäten der von dem einzelnen Leuchtdioden empfangenen Lichtanteile im wesentlichen gleich. In Figur 3 ist eine normierte Intensität gezeigt.

Versuche haben ergeben, daß bei geringen Schichtdicken von etwa 1 mm das Verhältnis der Intensitäten der von den äußeren (A) Leuchtdioden 410, 420, 430 stammenden Lichtanteile zu den von der inneren (I) Leuchtdiode 200 stammenden Lichtanteile etwa 1 : 65, und bei einer Schichtdicke von ca. 28 mm 2 : 1 beträgt. Dieser große Bereich der Variation der Intensitätsverhältnisse erlaubt eine sehr genaue Bestimmung der Schichtdicke.

Wie in Figur 3 weiter gezeigt ist, beträgt die Länge eines Meßzyklus 100 Mikrosekunden. Dadurch können äußere Einflüsse beispielsweise durch Bewegen der Sonde relativ zu der zu bestimmenden Schicht minimiert werden.

Die von dem Lichtsensor 100 aufgenommenen Meßwerte werden dem Rechner 20 zugeführt. Der Rechner 20 nimmt eine Digitalisierung der in analoger Form vorliegenden Meßwerte vor und berechnet die genannten Intensitätsverhältnisse.

Zur weiteren Steigerung der Genauigkeit bzw. zur Minimierung äußerer Einflüsse auf das Ergebnis speichert der Rechner jeweils die Ergebnisse von 200 Meßzyklen und nimmt sodann eine Mittelwertbildung vor, bevor aus dem gebildeten Mittelwert dann die Schichtdicke bestimmt wird.

Die ermittelte Schichtdicke kann auf einem Monitor angezeigt werden.

Bei dem in Figur 1 gezeigten Ausführungsbeispiel ist jedoch (zusätzlich oder alternativ zu dem Monitor) das Kartenschreib/Lesegerät 30 vorgesehen. In das Gerät können Speicherkarten, beispielsweise Chipkarten oder Magnetkarten, eingegeben werden, um die für die Fettschichtdicken ermittelten Werte zu speichern.

Dadurch ergibt sich die Möglichkeit, Aussagen über die Veränderung der Fettschichten im Verlaufe einer eventuellen Therapie zu machen, um die Therapie an dem erzielten Ergebnis auszurichten.

Wie bereits erwähnt, kann die oben beschriebene Vorrichtung Teil einer Vorrichtung zum Ermitteln von Daten betreffend den Gesundheitszustand eines Säugetieres, insbesondere eines Menschen sein, wobei noch andere Meßeinrichtungen vorgesehen sein können. Eine derartige Vorrichtung, welche gleichzeitig mehrere Parameter bezüglich des Gesundheitszustands insbesondere von Menschen aufnehmen kann, ist insbesondere in Kurkliniken, aber auch in Praxen von Allgemeinmedizinern und in Krankenhäusern einzusetzen.

Insbesondere dann, wenn die oben beschriebene Einrichtung zum Bestimmen des Körperbaus insbesondere eines Menschen, ein Blutdruckmeßgerät und eine Waage in einer einzigen Vorrichtung kombiniert sind, wobei selbstverständlich auch Speichereinrichtungen, insbesondere zur Speicherung der Daten auf Speicherkarten, vorgesehen sind, kann eine Therapie unter Berücksichtigung aller entscheidenden Parameter durchgeführt werden.

Dabei kann auf besonders einfache Weise die jeweilige Reaktion des Körpers auf die Therapie mit einbezogen werden, so daß eine Einbeziehung der genannten Reaktion im Sinne einer Regelung erfolgen kann.

## Patentansprüche

1. Vorrichtung zur Bestimmung der Dicke einer Fettschicht mit zumindest zwei voneinander beabstandeten Lichtquellen (200,300,410,420,430) zur Lichtbeaufschlagung der Fettschicht und mit zumindest einem Lichtsensor (100) zur Aufnahme des gestreuten Lichtes, an welchen Lichtsensor (100) eine Auswerteeinrichtung (20) angeschlossen ist, welche Vorrichtung an die zu untersuchende Fettschicht anlegbar ist, dadurch gekennzeichnet, daß die anlegbaren Lichtquellen (200,300,410,420,430) für vorgegebene Beleuchtungszeiten nacheinander ein- und ausschaltbar sind, daß der Lichtsensor mit jeweils unterschiedlicher Entfernung zu den mindestens zwei Lichtquellen (200,300,410,420,430) angeordnet ist, und daß an den Lichtsensor (100) die Auswerteeinrichtung (20) zum Ermitteln des der Verhältnissese der Intensität-en der von dem Lichtsensor (100) empfangenen Streulichtmengen angeschlossen ist, wobei die Dicke der Fettschicht aus den Verhältnissen der von dem Lichtsensor (100) empfangenen Streulichtintensitäten des jeweils bei der Beleuchtung der Fettschicht an den verschiedenen Orten ausgesandten Lichtes bestimmbar ist.

2. Vorrichtung nach Anspruch 1, dadurch gekennzeichnet, daß fünf Lichtquellen (200,300,410,420,430) vorhanden sind, von denen drei (200,300,420) in einer Reihe mit dem Lichtsensor (100) und zwei (410,430) auf einem um den Lichtsensor (100) herum auf einem durch die am weitesten von dem Lichtsensor (100) entfernte (420) der drei in Reihe liegenden Lichtquellen (200,300,420) verlaufenden Kreisbogen liegen.

3. Vorrichtung nach Anspruch 1 oder 2, dadurch gekennzeichnet, daß die Lichtquellen (200,300,410,420,430) Leuchtdioden sind.

4. Vorrichtung nach einem der Ansprüche 1 bis 3, dadurch gekennzeichnet, daß jeweils vor und nach einer Beleuchtungszeitspanne eine Zeitspanne ohne Beleuchtung bzw. eine Dunkelzeit eingeschaltet ist.

5. Vorrichtung nach einem der Ansprüche 1 bis 4, dadurch gekennzeichnet, daß ein Zyklus des Beleuchtens der Fettschicht an den verschiedenen Orten nacheinander etwa 100 µs lang ist.

6. Vorrichtung nach einem der Ansprüche 1 bis 5, dadurch gekennzeichnet, daß die Auswerte einrichtung (20) die jeweils ermittelten Verhältnisse speichert und nach jeweils 200 Meßzyklen aus den gespeicherten Verhältnissen einen Mittelwert bildet.

7. Vorrichtung nach einem der Ansprüche 1 bis 6, dadurch gekennzeichnet, daß die ermittelten Schichtdicken in der Auswerte einrichtung (20), z.B. auf einer Speicherkarte, speicherbar sind.

8. Vorrichtung nach einem der Ansprüche 1 bis 7, dadurch gekennzeichnet, daß an die Vorrichtung ein Spirometer und/oder ein Blutdruckmeßgerät und/oder eine Waage und/oder eine Einrichtung zum Bestimmen des Körperbaus, d.h. der Größe, der Schulterbreite, der Hüftbreite sowie der Brusttiefe des Körpers, angeschlossen sind.

## Claims

1. A device for detecting the thickness of a layer of fat having at least two mutually spaced light sources (200, 300, 410,420, 430) for exposing the layer of fat to light and at least one light sensor (100) for receiving the scattered light, to which light sensor (100) an evaluation means (20) is connected, said device being applicable to the layer of fat to be examined, characterized in that the applicable light sources (200, 300, 410, 420,430) are formed to be consecutively switched on and off for predetermined radiation periods, that the light sensor is disposed at respectively different distance from the at least two light sources (200, 300, 410, 420, 430) and that the evaluation means (20) is connected to the light sensor (100) for determining the ratio(s) of intensity (intensities) of the amounts of scattered light received by the light sensor (100), the thickness of the layer of fat being determinable on the basis of the ratios of the scattered light intensities received by the light sensor (100) of the respective light emitted in various locations for lighting the layer of fat.

2. The device according to claim 1, characterized in that five light sources (200, 300, 410, 420, 430) are provided of which three (200, 300, 420) are aligned with the light sensor (100) and two (410, 430) are disposed on a circular arc extending around the light sensor (100) through the light source (420) remotest of the three aligned light sources (200, 300, 420) from the light sensor (100).

3. The device according to claim 1 or 2, characterized in that the light sources (200, 300, 410, 420, 430) are light-emitting diodes.

4. The device according to any one of the claims 1 to 3, characterized in that each lighting period is preceded and followed by a period without lighting or an interval of darkness.

5. The device according to any one of the claims 1 to 4, characterized in that a cylcle of lighting the layer of fat in the various locations lasts about 100 µs.

6. The device according to any one of the claims 1 to 5, characterized in that the evaluation means (20) stores the respective determined ratios and forms a mean value from the stored ratios after each 200 measuring cycles.

7. The device according to any one of the claims 1 to 6, characterized in that the determined layer thicknesses are storable in the evaluation means (20), for instance on a memory card .

8. The device according to any one of the claims 1 to 7, characterized in that a spirometer and/or a blood pressure gauge and/or a scale and/or a means for determining the body structure, i.e. the size, shoulder width, hip width and chest depth of the body, are attached to the device.

## Revendications

1. Dispositif pour détecter l'épaisseur d'une couche de graisse comprenant au moins deux sources lumineuses (200,300, 410,420,430) espacées l'une de l'autre pour émettre de la lumière à la couche de graisse, et au moins un photodétecteur (100) pour recevoir la lumière dispersée, auquel photodétecteur (100) un moyen d'évaluation est relié, le dispositif pouvant être mis en contact contre la couche de graisse à examiner, caractérisé en ce que les sources lumineuses (200,300,410,420, 430) pouvant être mis en contact peuvent être mis successivement dans et hors circuit pour des périodes d'illumination prédonnées, en ce que le photodétecteur est disposé à distances différents à chacune des au moins deux sources lumineuses (200, 300,410,420,430), et que le un moyen d'évaluation (20) est relié au photodétecteur (100) pour détecter le(s) rapport(s) d'intensité des quantités, l'épaisseur de la couche de graisse pouvant être déterminée des rapports des intensités de la lumière dispersée qui sont reçues par le photodétecteur (100) de la Lumière émise de places différents pour illuminer la couche de graisse.

2. Dispositif selon la revendication 1, caractérisé en ce qu'il y a cinq sources lumineuses (200,300,410,420,430) dont trois (200,300,420) sont disposées dans une enfilade avec le photodétecteur (100) et dont deux (410,430) sont disposées autour du photodétecteur (100) à un arc de cercle passant par la source lumineuse (420) la plus éloignée du photodétecteur (100) des sources lumineuses (200,300,420) disposées dans l'enfilade.

3. Dispositif selon la revendication 1 ou 2, caractérisé en ce que les sources lumineuses (200,300,410,420,430) sont des diodes lumineuses.

4. Dispositif selon l'une des revendications 1 à 3, caractérisé en ce qu'avant et après chaque période d'illumination une période sans illumination ou période d'obscurité est intercalée.

5. Dispositif selon l'une des revendications 1 à 4, caractérisé en ce qu'un cycle d'illumination de la couche de graisse aux places différents dure successivement environ 100 s.

6. Dispositif selon l'une des revendications 1 à 5, caractérisé en ce que le moyen d'évaluation (20) mémorise chacun des rapports déterminés et forme la valeur moyenne des rapports mémorisés après chaques 200 cycles de mesure.

7. Dispositif selon l'une des revendications 1 à 6, caractérisé en ce que les épaisseurs de couche déterminées peuvent être mémorisées dans le moyen d'évaluation (20), par exemple sur une carte de mémorisation.

8. Dispositif selon l'une des revendications 1 à 7, caractérisé en ce qu'un spiromètre et/ou un appareil mesureur de la tension artérielle et/ou une balance et/ou un appareil pour déterminer la complexion, c'est à dire la taille, la carrure, la largeur des hanches ainsi que la profondeur pectorale, sont reliés au dispositif.
